# EUROPEAN PATENT SPECIFICATION

(11) **EP 2 126 036 B1**
(45) Date of publication and mention of the grant of the patent: **09.08.2023**
(21) Application number: 08701690.3
(22) Date of filing: 04.01.2008
(51) Int. Cl.: C12M 1/00

(54) **BIOTECHNICAL AND MICROBIOLOGICAL PRODUCTION METHOD AND EQUIPMENT**
BIOTECHNISCHES UND MIKROBIOLOGISCHES HERSTELLUNGSVERFAHREN UND -GERÄT
PROCÉDÉ ET ÉQUIPEMENT DE PRODUCTION BIOTECHNIQUE ET MICROBIOLOGIQUE

(30) Priority: 04.01.2007 FI 20070008
(43) Date of publication of application: 02.12.2009
(73) Proprietor: Hakalehto, Eino, Elias, 70110 Kuopio (FI)
(72) Inventor: Hakalehto, Eino, Elias, 70110 Kuopio (FI)
(74) Representative: Tomkinson, Alexandra
(86) International application number: PCT/FI2008/000001
(87) International publication number: WO 2008/081082

(56) References cited:
- EP-B1- 0 994 181
- WO-A1-00/29604
- WO-A1-99/23243
- WO-A1-03/055606
- WO-A2-2005/034613
- WO-A2-2008/088371
- DE-A1- 10 224 665
- US-A- 3 919 053
- US-A- 4 350 588
- US-A1- 2005 040 107
- HAKALEHTO E. ET AL.: 'Aerobic and anaerobic growth modes and expression of type 1 fimbriae in Salmonella' PATHOPHYSIOLOGY vol. 14, no. 1, 31 May 2007, pages 61 - 69, XP022049664

## Description

### Background for the invention

In utilizing and studying microbes in industry or medicine or in environmental cleaning, the used microbiological and bacteriological methods in most cases call for the culturing of cells or equivalent in a nutrient substrate before being able to show them, clarifying the effects of their action or getting to a desired production result. This production result may be a cell growth, e.g. for feed or protein, or the formation of one or several desired metabolic products. These products can be in liquid or gas form or they can be separated from a production liquid into solid form by precipitation. The production result of a microbiological reaction can also be the cleaning of environment, like soil or water, or the elimination of a harmful substance from an organism.

Nutrient substrates contain vital nutrients for microbes and they are designed to be as suitable as possible for the microbes and other organisms to be studied. Plant and animal cells have been started to generally be cultivated with similar methods as microbes.

Conventionally, nutrient substrates are divided into general nutrient substrates and selective nutrient substrates. In the former many different microbes can widely be cultivated whereas selective nutrient substrates choose or enrich selective species or strains. Studied microbes may be for example single cell bacteria or yeasts, or filamentous molds, or algae or protozoa.

Microbe cultivations and studies may be related to projects for example in medicine, health care, pharmaceutical industry, food, chemistry or cosmetics industries or forestry industry. They may also be part of the control of building mold damage or the follow-up of environmental condition to find out the quality of water, air or other environmental quality.

Normally these cultivations take place in thermostatically controlled cabinets (thermal cabinets or incubators) or rooms, which thermostated cabinets are located in laboratories or equivalent. Often there is need to collect microbe samples or equivalent in the field or as part of field experiments or on site for example in production processes, different locations in hospitals or canteens. Large quantities of microbe cells are generally tried to be cultivated in different kinds of bioreactors, fermentors. Microbiological production reactions often also take place in these. In studies performed by Finnofiag Oy (Kuopio and Siilinjarvi, Finland), it has been shown that microbes can efficiently be cultivated in controlled environments in a cultivation case in a way that air or gas is led into the sampling and cultivation syringes in the case (Finnish patent no. 106561).

In the biotechnical industry fermentation has conventionally been used for cultivating microbes or carrying out a production method by them. Although "fermentation" has originally meant anaerobic fermentation, this concept nowadays includes all microbe action that is carried out in bioreactors. This action may happen just as well in the presence of oxygen, aerobically, or without oxygen, anaerobically. In this application the term "reactor" means bioreactor.

In fermentation a device in which the biotechnical or microbiological reaction happens is called a bioreactor or fermentor. Originally it was used for microbe reactions like the reactions needed in the production of microbe cell mass, metabolism products, enzymes, antibiotics or other products carried out by bacteria, yeasts or molds. Later also euchariotic organisms, plant or animal cells have been used in these reactions. A bioreaction or fermentation can be carried out except for forming a product, also to clean a material, for example by breaking up toxins or by consuming nutrients. Thus all kinds of biological refineries are also bioreactors. The form of a bioreactor may be a barrel-like container or pipe or tube or a tray-like surface or a bottle or can shaped container or a basin or tank or equivalent. Essential is that a product or products are formed using a biocatalyst or a desired service, like cleaning water in a biological refinery or the binding of nitrogen to soil using nitrogen-binding bacteria, is carried out. The form of a bioreactor may partly be round as in Finnish patent no FI 115967 or like an injection syringe as in Finnish patent no. FI 106561.

One important form of a bioreactor is the cylindrical form. It contains a mixing device, normally some kind of a propeller. In this case the reactor, i.e. fermentor is called also with the term STR (stirred tank reactor). If the cultivation is continuous the reactors name can be CSTR (continuous stirred tank reactor). In this case fresh nutrient substrates are added to the reactor container continuously and correspondingly reaction liquid is removed continuously. When cultivating aerobic microbes the nutrient substrate, i.e. liquid in a reactor is normally aerated by pumping air into it for example by a tube pump. If this mixing of liquid nutrient substrates takes place by using air and not for example mechanically, the reactor is called an "air lift" fermentor. When a biocatalyst, which can be cells, cell tissue, mycelium growth of a microbial mushroom (mold) or bacteria (e.g.hyphal forms), cell parts or structures or enzymes, is not situated freely in the reactor liquid but is attached to some carrier material in immobilized form and air is led to the reactor and reactor liquid, a fluidized bed fermentor is formed.

If a hollow structure, for example a tube is used as a reactor, one can talk of a hollow fiber type of a fermentor. All in all the structure of the reactor may be any solution that is applicable to the bioreaction to be carried out. The volume of the reactor/fermentor may vary according to the task. Normally the smallest reactors are just one liter laboratory fermentors, the biggest can be large tanks with capacity of hundreds of cubic metres. Also water refinery basins are bioreactors as well as basins in which single cell proteins are produced under sun light with the help of algae.

In aerobic fermentation the basis is to prevent gas that contains oxygen of getting into the bioreactor. The mixing is normally done mechanically. As a result of their metabolism, anaerobic bacteria normally form gases and thus excess pressure into the reactor which helps its content stay anaerobic. Because of gases and liquid pressures, the movements of these or the mechanical operation of the device, such as the movement of stirrers, different so-called shear forces are formed in the reactor. These cause strain to the microbes or cells or equivalent in the liquid, suspension, reactor mixture, culture or equivalent in the reactor, which can also be called environmental stress. In many cases it has been established that shear forces promote the attachment of microbes or cells or equivalent onto surfaces as biofilms. By environmental stress one can also mean the sum of all environmental effects which decreases the viability or functionality of microbes or cells or equivalent in a reactor or culture.

When nutrients and reaction products are continuously added and removed from the reactor during the reaction, the bioreactor is called continuously operating and the corresponding single cultivation batch cultivation or batch culture. The continuous operation of the reaction often improves its adjustability and the formation of products, their yield and productivity. Organisms, that possibly can be used as biocatalysts in the reactor, can be aerobic, or microaerophilic, which need only small amounts of oxygen, or facultatively anaerobic, that can grow and metabolise both with the presence of oxygen and without it, or oblicately anaerobic, which do not tolerate oxygen. Facultatively anaerobes are among others enteric bacteria, for example the bacteria from the genera Escherichia, Salmonella, Klebsiella, Citrobacter, Aerobacter, Serratia and Enterobacter. Of these, for example Escherichia species anaerobically form especially lactic acid, acetic acid and succinic acid, ethanol and formic acid (or hydrogen and carbon dioxide) ( so called mixed acid fermentation), whereas for example Enterobacter species produce additionally 2,3- butanediol and more ethanol and considerably less acid metabolic products (so called butanediol fermentation). Facultatively anaerobic bacteria also include pathogenic strains such as Salmonella and Vibrio cholerae strains.

In many biotechnical reactions microbe cells and other cells are produced in different conditions compared with the conditions in which the actual formation of the product optimally happens. Correspondingly, the pretreatment of raw materials, such as enzymatic hydrolysis, can require different conditions than the actual production reaction in which the raw material is worked up into a final product such as biofuel, solvent, acid, basic chemical or other equivalent product.

Microbes can be cultured as pure cultures, in which case the entire microbe population represents the same microbe strain, or as mixed cultures. In the latter option one can exploit the influence that different microbes together or in cooperation can achieve. In this case the environmental conditions have to be adjusted so that the desired production or product forming conditions are realized.

EP09941818 discloses a device for the measurement of interactions of the metabolism of aerobic and anaerobic cell systems comprising at least one co-culturing chamber which is separated by a permeable, cell impermeable separating wall into first and second compartments. Gas supply means supplies the first chamber compartment with aerobic gas and the second chamber compartment with anaerobic gas. Wherein the aerobic cells and anaerobic cells generate metabolic products which can pass through the permeable separating wall into the other compartment and can be analyzed.

WO99/23243 discloses a method for enriching and examining microbiological samples wherein microbes are brought into a syringe and a gas or gas mixture is conducted into the syringe.

WO03/055606 discloses portable microbe enrichment equipment including a case containing sample containers and replaceable insulating padding or lining material. The sample containers are controlled for measurements and temperature. Gas can be fed into the sample containers.

US3919053 discloses a specimen culturing assembly for cultivation of a specimen simultaneously under both anaerobic and aerobic environmental conditions. The assembly comprises an aerobic container and an aerobic container releasably locked or clamped together. A special valve and tube allows a technician to alternately direct the fluid containing the micro-organisms into the aerobic container and then into the anaerobic container.

WO00/29604 discloses a process for the production of biological products by microorganisms, whereby the microorganisms are selected that are capable of utilizing oxygen and an alternative oxidant source other than oxygen for cellular respiration.

US2005/0040107 discloses a single vessel multi-zone wastewater bio-treatment system. An aerobic zone is provided in the upper central portion of the vessel and air is fed into this zone for oxygenation and creating an upflow. An annularly disposed anoxic zone is created about said aerobic zone causing the upflow from the aerobic zone to produce a downflow in the anoxic zone, thereby causing a portion of the downflow from the anoxic zone to pass into the upflow of the aerobic zone.

### Description of the invention

According to a first aspect of the present invention there is provided a method for carrying out a fermentation in an air-lift bioreactor according to claim 1.

According to a further aspect of the present invention there is provided an air-lift bioreactor for carrying out a fermentation according to any of claims 1-14.

In a bioreactor it can be advantageous to cultivate in one part in aerobic conditions a specific microbe population and in another part anaerobically either the same or a specific other microbe population. With the help of this approach, in the aerobic part of the bioreactor one can reach maximum yield for example in the case of cell growth, and cells or substances suitable for microbe nutrients that have preparatively been broken down by cells, move into the anaerobic part. In the anaerobic parts, metabolic products that can start to hinder the production or the continuous formation of the product through different genetic and biochemical adjustment mechanisms, either move to the aerobic part or are collected from the bioreactor.

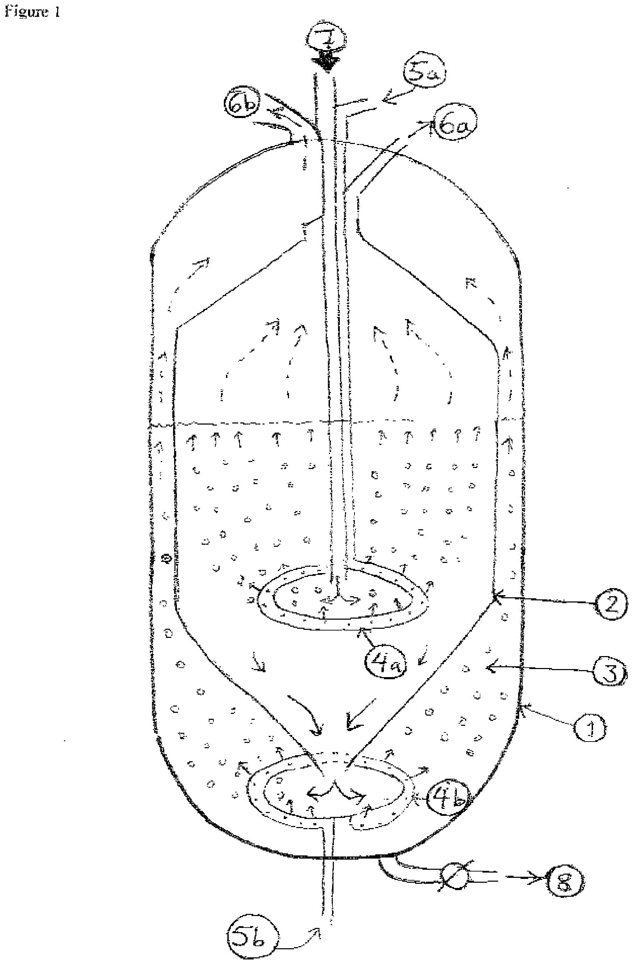

To exploit the observation that the anaerobic growth and metabolism of microbes has, in our test set-ups, been comparable in speed to their aerobic growth and metabolism, we have designed a bioreactor solution, which is described in this patent application (Fig. 1). A special feature in this is that gas is led into different parts of the reactor liquid or reactor mixture in the reactor and that the oxygen content can be 0 - 100 %. Further: in order to gain the best result, different gas or gas mixture are led to different parts of the reactor.

As gases that are led into the reactor liquid or reactor mixture bubble they cause liquid and gas phase interfaces. The forming of these, speed up the movement of materials. Thus on the one hand, one gets nutrients quickly for the cells to use and on the other hand waste products are moved away from the cells. Taking these considerations into account and because optimal conditions for cell growth, cell respiration product forming, enzymatic reactions, fermentation reactions and the breaking up of different substances etc. can be effectuated in different parts of the liquid or mixture, offers the method and equipment according to the present invention a possibility of utilizing in a new and more efficient way all of the biocatalyst potential that microbe cells or equivalent have to offer. Additionally essential is that the flow of gases in different parts of the reactor may each time be adjusted directly according to the available measurement results in order to gain the best outcome of the process.

Thus for example the cultivation of Klebsiella sp. strain that forms butanediol can be carried out in the reactor's aerobic part after which the cells that drift to the microaerobic or anaerobic parts and can carry out the formation of the actual product, 2,3-butanediol. With this approach it is possible in some cases to avoid the need for immobilizing the biocatalyst, for example, which in part produces cost savings. When returning back to the aerobic area, the Klebsiella cells of this example can continue their multiplication, and thus increase the biocatalyst formation.

In order to optimize the usability of different reactor solutions, different parts of the reactor have to be screened by measurements. These measurements may include e.g. pH, pO₂, pCO₂, optical density, analyte concentrates etc. It is of particular importance that these pieces of information allow correct adjustments of the gas flows to different parts of the reactor.

The method and apparatus according to the present invention are particularly specifically suitable for exploiting the facultatively anaerobic bacteria. For example, by their help it is possible to produce organic acids, alcohols or 2,3-butanediol for the needs of chemical, food or polymer industries. These organisms are able to ferment anaerobically or microaerobically by using mixed acid fermentation or butanediol fermentation route. Bacteria of the genera Klebsiella or Enterobacter, for instance, having the capability to fix atmospheric nitrogen, can utilize the latter metabolic pathway. When fixing atmospheric nitrogen in the aerobic part of the reactor, it can be exploited in anaerobic fermentation as the bacteria that act as biocatalysts move to the anaerobic part of the reactor. In this patent application the words reactor, bioreactor and fermentor have the same meaning.

The method according to the present invention could be exploited also when different microbes are cultivated as mixed cultures. By creating different gas exchange conditions into different parts of the fermentor it is possible to achieve optimal conditions for the product formation into areas where enhanced microbe function is required.

To exploit the present invention, one advantageous bioreactor model is presented in Figure 1. When using it, two or more gases are directed via two or more routes into the reactor chamber (1) or basin or equivalent, in such a way that part of the gases are aerobic (contain oxygen) (4a, 5a) and another part are anaerobic (without oxygen) (4b, 5b). This is especially suitable for cultivating and exploiting facultatively anaerobic bacteria but also for the cultivation and utilization of many other microbes, such as some moulds.

The gases are led either entirely or in part into the solution, suspension, reaction mixture, culture or equivalent (3). The bioreactor contains different compartments and has impermeable or partially permeable middle walls in between them. The exhaust gases coming out from the bioreactor or the cultivation chamber, and with them liberated liquids for example in aerosol form, or solid substances, that may be for example as particles (6a, 6b), are collected as usable products. The products are collected from the solution, suspension, reaction mixture, culture or equivalent (8), which is removed from the reactor.

The conditions inside the reactor are adjusted by the controlling unit according to the measurement data from the outgoing substance concentrations or characteristics obtained by measurements based on sensor data. The nutrients and additives can be fed advantageously into the aerobic reactor first through the inlet opening (7), from where they, for example by gravitation, settle down into the anaerobic part of the reactor. More than one inlet for nutrients or raw materials may also be used.

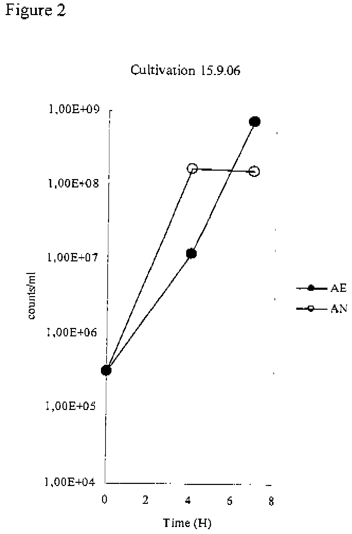

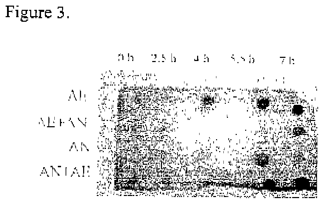

Example 1. One example, in this case of the different activities of one microbe Salmonella enterica serovar Enteritidis in aerobic and anaerobic conditions, can be seen in the growth curve (Figure 2) and dot blot immunoassay picture (Figure 3). The bacterium in question (strain IHS 59813) was cultivated in a PMEU equipment (Portable Microbe Enrichment Unit, Finnoflag Oy, Kuopio and Siilinjarvi, Finland). Before inoculating the cultivation syringes of the enrichment unit, the bacterial inoculant strain was made younger into a culture in TYG (Tryptone- Yeast extract- Glucose) broth 24 hours before the onset of the experiment. Salmonella culture was taken into the TYG medium in the cultivation syringes as a final concentration of 10.000- 100.000 CFU/ml by visual estimation.

The cultivations were carried out as follows:
Syringe 1: Aerobic culture in the PMEU enrichment equipment, air flow 100%, temperature +37[deg.]C for 2 hours, after that +40°C until the end of the experiment.
Syringe 2: Aerobic culture in the PMEU enrichment equipment, air flow 100%, temperature +37[deg.]C for 2 hours, after that +40°C, after 3 hours from the onset of the cultivation the syringe switched into another PMEU equipment for anaerobic cultivation.
Syringe 3: Anaerobic culture using nitrogen gas in the PMEU enrichment equipment, gas flow adjusted visually to the same as in the aerobic case, temperature +37[deg.]C for 2 hours, after that +40[deg.]C until the end of the experiment.
Syringe 4: Anaerobic culture using nitrogen gas in the PMEU enrichment equipment, gas flow adjusted visually to the same as in the aerobic case, temperature +37[deg.]C for 2 hours, after that +40[deg.]C, after 3 hours from the onset of the cultivation the syringe switched into another PMEU equipment for aerobic cultivation

### Samples collected during cultivation

O h to plate culture from the bacterial suspension applied to the syringes, and from each syringe to dot blot analysis
2.5 h to dot blot analysis
4 h to plate culture and dot blot analysis
5.5 h to dot blot analysis
7 h to plate culture and dot blot analysis

### Plate cultures:

Samples were diluted to sterile water and inoculated to TYG plates. The plates were incubated in an incubator chamber for 24 hours at +40[deg.]C and then the colonies we re

20 counted. The growth curves were drawn accordingly (Figure 2). In this experiment, against general belief that anaerobic metabolism would be slower than aerobic, with the PMEU equipment, a faster initial bacterial growth was achieved anaerobically than by aerobic metabolism (0-4 h). After that the growth attenuated probably due to the exhaustion of the available nutritive substances. Therefore, already after 8 hours the production of bacterial biomass is on a higher level in the aerobic PMEU cultivation syringe than in the anaerobic one.

### Dot blot analysis

The samples from different time points were applied to nitrocellulose filter paper strip. The strip was allowed to dry up, after which it was preserved in a refrigerator (7 days) until the analysis.

The strip was moved for blocking into the BSA-TBS -solution, where it was kept in a shaker for 1 hour. After that the strip was incubated in an antibody solution (H463 12.3.98, dilution 1:70 in 1 x TBS) for 1 hour. After the antibody bath the strip was washed 3 x 10 min in a washing solution (1.5 % milk powder/ 0.5 % Tween in TBS).

Then the strip was incubated in the secondary antibody solution (AP-Goat Anti-Rabbit IgG, dilution 1:1000 in TBS) for 1 hour. The washes were repeated 2 x 10 min in the washing solution and 1 × 10 min in the Afos buffer solution. In the end the strip was stained in the staining solution (NTB+BCIP in Afos buffer solution). The stained strip is seen in Figure 3.

Aerobically grown bacterial samples from different time points (AE) and the anaerobic samples from different time points (AN) indicate different fashions for the formation of the fimbrial attachment threads in these different conditions. In the anaerobic cultivation the maximal fimbrial synthesis took place at 5.5 h with their expression attenuating after that, whereas in the aerobic process the production of the protein in question remained on a high level also after that. When the culture was started aerobically and switched to the anaerobic mode (AE+ AN), or vice versa (AN+ AE), the production was delayed when the conditions changed.

When the plate results from the PMEU cultivations were studied, it was found out that the anaerobic cultivation had produced faster cell number increase between 0-4 hours, which was a surprising result, because aerobic metabolism has generally been considered faster than anaerobic (Figure 2). This common belief is based on the cellular production of two ATP moles per one mole of glucose in for instance homolactic fermentation, whereas in aerobic metabolism they generate 28 moles. On the basis of the research behind the present invention it seems to be so, that in the anaerobic fermentation the availability of the nutrients and the removal of diffusion limitations could replace the shortness of the metabolic route.

Example 2. In a similar way as in the example 1 Escherichia coli (ATCC 25922) and Klebsiella mobilis (ATCC 13048) bacterial strains were studied anaerobically, microaerobically and aerobically with the PMEU equipment. It was found out that the latter strain formed 2,3-butanediol already after some hours of cultivation, and in such a way that the butanediol formation continued also after the bacterial growth had become slower. In microaerobic cultures a gas mixture of 5% 02, 10% CO2, and 85% N2 was used. The growth produced in the aerobic and microaerobic pure cultures is seen in Table 1. In Table 2 the aerobic growth produced by the Escherichia coli (ATCC 25922) and Klebsiella mobilis (ATCC 13048) mixed cultures is presented. An interesting observation was that when in both experiments TYG (Tryptone- Yeast extract- Glucose) broth was used as growth medium, both the pure and mixed cultures gave equal yields for both bacterial strains. This indicated that these bacterial strains did not compete on the nutrients in the given circumstances. Nitrogen gas was used in the anaerobic cultivation. The plate cultivations were carried out as described in Example 1, the growth medium being Chromagar TM Orientation Medium (Becton Dickinson, USA), pH measurements were carried out with Orion Model 420A pH meter (Thermo Electron Corporation, USA).

## Claims

1. A method for carrying out a fermentation in an air-lift bioreactor by leading two or more different gases into the air-lift bioreactor so that the bioreactor has different compartments which have impermeable or partially permeable middle walls between them to form an aerobic part and an anaerobic part, wherein the aerobic part is located inside the anaerobic part of the bioreactor; **characterized in that** the different gases are led into the different parts of the bioreactor, the different gases including:
a) an oxygen-free gas mixture (4b, 5b) which is led into the anaerobic part of the bioreactor, wherein the gas mixture contains an inert gas for flushing out or for collecting the evaporating microbial or other cellular reaction products or waste substances or other evaporating substances, in order to increase the anaerobic or microaerobic fermentation yield or productivity with said inert gas, and that
b) an air or other oxygen-containing gas (4a, 5a) which is led into the aerobic part of the bioreactor, wherein the gases led into the reactor cause interphases between the liquid and gas phases, and
wherein the flow of gases led into the bioreactor is adjusted using a control unit, which enables adjusting the conditions inside of the bioreactor according to the sensor measurement data obtained from the concentrations or characteristics of the substances outgoing from the bioreactor, and
wherein mixing of the reagents in the bioreactor is carried out by air, oxygen free gas or oxygen containing gas, avoiding mechanical stirring.

2. The method according to claim 1, **characterized in that** the inert gas is nitrogen.

3. The method according to claim 1 or 2, **characterized in that** all gases (5a, 5b, 4a, 4b) led to the bioreactor or a part of them are sterilized.

4. The method according to any one of claims 1-3, **characterized in that** the gases (5a, 5b, 4a, 4b) led to the reactor form bubbles into the solution, suspension, reaction mixture, culture or equivalent (3) included in the bioreactor.

5. The method according to any one of claims 1-4, **characterized in that** the substances liberated in a gaseous form from the solution, suspension, reaction mixture, culture or equivalent (3) are led out as a uniform gas mixture, whose composition may vary.

6. The method according to any one of claims 1-5, **characterized in that** the substances liberated in a gaseous form from the solution, suspension, reaction mixture, culture or equivalent (3) are led out as two or more gas mixtures with different compositions using two or more different routes.

7. The method according to claim 5 or 6, **characterized in that** gaseous, liquid or solid substances are collected for exploitation from the one or more gas flows out of the bioreactor.

8. The method according to any one of claims 1-7 **characterized in that** the physical conditions, temperature or pressure, of the gases (5a, 5b, 4a, 4b) led into the bioreactor from two or more sources are different when compared to each other.

9. The method according to any one of claims 1-8, **characterized in that** substances are added into or taken out from the solution, suspension, reaction mixture, culture or equivalent (3) continuously.

10. The method according to any one of claims 1-9, **characterized in that** bacteria are used as biocatalysts in the bioreactor.

11. The method according to any one of claims 1-10, **characterized in that** facultatively anaerobic bacteria are used as production organisms in the fermentation in the bioreactor.

12. The method according to claim 11, **characterized in that** enteric bacteria or enterobacteria are used as production organisms.

13. The method according to claim 12, **characterized in that** the production organisms belong to the genera *Klebsiella* or *Enterobacter* or any other genera using butanediol fermentation in their metabolism.

14. The method according to any one of claims 11-13, **characterized in that** the bacterium used as a production organism is capable of fixing the atmospheric nitrogen in the aerobic part of the bioreactor, and atmospheric nitrogen can thus be exploited also in the anaerobic fermentation.

15. An air-lift bioreactor for carrying out a fermentation according to any one of claims 1-14, the bioreactor comprises a reaction chamber (1) or basin, which is configured to have different compartments which have impermeable or partially permeable middle walls between them to provide an aerobic part of the bioreactor and an anaerobic part of the bioreactor, wherein the aerobic part is located inside the anaerobic part of the bioreactor; **characterized in that** the bioreactor is configured such that different gases are led into the different parts of the bioreactor, the different gases including a) an oxygen free gas mixture (4b, 5b) which is led into the anaerobic part of the bioreactor, and b) air or other oxygen containing gas (4a, 5a) which is led into the aerobic part of the bioreactor, and **in that** the bioreactor is connected to a control unit enabling adjustment of the conditions inside the bioreactor according to the sensor measurement data obtained from the concentrations or characteristics of the substances outgoing from the bioreactor, wherein the bioreactor does not contain mechanical means for stirring.

16. The apparatus according to claim 15, **characterized in that** the gases (5 a, 5 b , 4a, 4 b) are led entirely or partially into the solution, suspension, reaction mixture, culture or equivalent (3) in the bioreactor.

17. The apparatus according to any one of claims 15-16, **characterized in that** the outgoing gases from the bioreactor or the reaction chamber, and with them the liberated liquids, are collected as usable products.

18. The apparatus according to any one of claims 15-17, **characterized in that** the products are collected from the outgoing solution, suspension, reaction mixture, culture or equivalent (8) and removed from the bioreactor.

## Patentansprüche

1. Verfahren zum Durchführen einer Fermentation in einem Airlift-Bioreaktor durch Einleiten von zwei oder mehreren verschiedenen Gasen in den Airlift-Bioreaktor, so dass der Bioreaktor verschiedene Kompartimente aufweist, die undurchlässige oder teildurchlässige Mittelwände zwischen sich haben, um einen aeroben Teil und einen anaeroben Teil zu bilden, wobei sich der aerobe Teil innerhalb des anaeroben Teils des Bioreaktors befindet;
**dadurch gekennzeichnet, dass** die verschiedenen Gase in die verschiedenen Teile des Bioreaktors geleitet werden, wobei die verschiedenen Gase Folgendes beinhalten:
a) ein sauerstofffreies Gasgemisch (4b, 5b), das in den anaeroben Teil des Bioreaktors geleitet wird, wobei das Gasgemisch ein Inertgas zum Ausspülen oder zum Sammeln der verdampfenden mikrobiellen oder anderen zellulären Reaktionsprodukte oder Abfallsubstanzen oder anderer verdampfender Substanzen enthält, um die anaerobe oder mikroaerobe Fermentationsausbeute oder Produktivität mit dem genannten Inertgas zu erhöhen, und dadurch, dass
b) Luft oder ein anderes sauerstoffhaltiges Gas (4a, Sa), das in den aeroben Teil des Bioreaktors geleitet wird, wobei die in den Reaktor geleiteten Gase Interphasen zwischen der Flüssig- und der Gasphase verursachen, und
wobei der Fluss von in den Bioreaktor geleiteten Gasen mittels einer Steuereinheit eingestellt wird, die es ermöglicht, die Bedingungen innerhalb des Bioreaktors gemäß den Sensormessdaten einzustellen, die aus den Konzentrationen oder Eigenschaften der aus dem Bioreaktor austretenden Substanzen erhalten werden, und
wobei das Mischen der Reagenzien im Bioreaktor mit Luft, sauerstofffreiem oder sauerstoffhaltigem Gas unter Vermeidung von mechanischem Rühren erfolgt.

2. Verfahren nach Anspruch 1, **dadurch gekennzeichnet, dass** das Inertgas Stickstoff ist.

3. Verfahren nach Anspruch 1 oder 2, **dadurch gekennzeichnet, dass** alle in den Bioreaktor geleiteten Gase (5a, 5b, 4a, 4b) oder ein Teil davon sterilisiert werden.

4. Verfahren nach einem der Ansprüche 1-3, **dadurch gekennzeichnet, dass** die in den Reaktor geleiteten Gase (5a, 5b, 4a, 4b) in der im Bioreaktor enthaltenen Lösung, Suspension, Reaktionsmischung, Kultur oder einem Äquivalent (3) Blasen bilden.

5. Verfahren nach einem der Ansprüche 1-4, **dadurch gekennzeichnet, dass** die aus der Lösung, der Suspension, dem Reaktionsgemisch, der Kultur oder dem Äquivalent (3) gasförmig freigesetzten Substanzen als einheitliches Gasgemisch ausgeleitet werden, dessen Zusammensetzung variieren kann.

6. Verfahren nach einem der Ansprüche 1-5, **dadurch gekennzeichnet, dass** die aus der Lösung, der Suspension, dem Reaktionsmischung, der Kultur oder dem Äquivalent (3) gasförmig freigesetzten Substanzen als zwei oder mehrere Gasgemische mit unterschiedlicher Zusammensetzung auf zwei oder mehreren unterschiedlichen Wegen ausgeleitet werden.

7. Verfahren nach Anspruch 5 oder 6, **dadurch gekennzeichnet, dass** gasförmige, flüssige oder feste Substanzen aus den ein oder mehreren Gasströmen aus dem Bioreaktor zur Verwertung gesammelt werden.

8. Verfahren nach einem der Ansprüche 1-7, **dadurch gekennzeichnet, dass** die physikalischen Bedingungen, Temperatur oder Druck, der aus zwei oder mehr Quellen in den Bioreaktor geleiteten Gase (5a, 5b, 4a, 4b) im Vergleich zueinander unterschiedlich sind.

9. Verfahren nach einem der Ansprüche 1-8, **dadurch gekennzeichnet, dass** der Lösung, der Suspension, dem Reaktionsgemisch, der Kultur oder dem Äquivalent (3) kontinuierlich Substanzen zugeführt oder entnommen werden.

10. Verfahren nach einem der Ansprüche 1-9, **dadurch gekennzeichnet, dass** Bakterien als Biokatalysatoren in dem Bioreaktor eingesetzt werden.

11. Verfahren nach einem der Ansprüche 1-10, **dadurch gekennzeichnet, dass** fakultativ anaerobe Bakterien als Produktionsorganismen bei der Fermentation im Bioreaktor verwendet werden.

12. Verfahren nach Anspruch 11, **dadurch gekennzeichnet, dass** als Produktionsorganismen enterische Bakterien oder Enterobakterien verwendet werden.

13. Verfahren nach Anspruch 12, **dadurch gekennzeichnet, dass** die Produktionsorganismen zu den Gattungen *Klebsiella* oder *Enterobacter* oder zu anderen Gattungen gehören, die Butandiol-Fermentation in ihrem Stoffwechsel verwenden.

14. Verfahren nach einem der Ansprüche 11-13, **dadurch gekennzeichnet, dass** das als Produktionsorganismus verwendete Bakterium den atmosphärischen Stickstoff im aeroben Teil des Bioreaktors fixieren kann, so dass atmosphärischer Stickstoff auch bei der anaeroben Fermentation genutzt werden kann.

15. Airlift-Bioreaktor zur Durchführung einer Fermentation nach einem der Ansprüche 1-14, wobei der Bioreaktor ein(e) Reaktionskammer (1) oder Becken umfasst, die/das so konfiguriert ist, dass sie/es verschiedene Kompartimente aufweist, die undurchlässige oder teildurchlässige Mittelwände zwischen sich aufweisen, um einen aeroben Teil des Bioreaktors und einen anaeroben Teil des Bioreaktors bereitzustellen, wobei sich der aerobe Teil innerhalb des anaeroben Teils des Bioreaktors befindet; **dadurch gekennzeichnet, dass** der Bioreaktor so konfiguriert ist, dass verschiedene Gase in die verschiedenen Teile des Bioreaktors geleitet werden, wobei die verschiedenen Gase a) ein sauerstofffreies Gasgemisch (4b, 5b), das in den anaeroben Teil des Bioreaktors geleitet wird, und b) Luft oder ein anderes sauerstoffhaltiges Gas (4a, Sa), das in den aeroben Teil des Bioreaktors geleitet wird, umfassen, und dadurch, dass der Bioreaktor mit einer Steuereinheit verbunden ist, die es ermöglicht, die Bedingungen im Inneren des Bioreaktors gemäß den Sensormessdaten einzustellen, die aus den Konzentrationen oder Eigenschaften der aus dem Bioreaktor austretenden Substanzen gewonnen werden, wobei der Bioreaktor keine mechanischen Mittel zum Rühren enthält.

16. Vorrichtung nach Anspruch 15, **dadurch gekennzeichnet, dass** die Gase (5a, 5b, 4a, 4b) ganz oder teilweise in die Lösung, Suspension, Reaktionsmischung, Kultur oder ein Äquivalent (3) im Bioreaktor geleitet werden.

17. Vorrichtung nach einem der Ansprüche 15-16, **dadurch gekennzeichnet, dass** die aus dem Bioreaktor oder der Reaktionskammer austretenden Gase und damit die freigesetzten Flüssigkeiten als verwertbare Produkte gesammelt werden.

18. Vorrichtung nach einem der Ansprüche 15-17, **dadurch gekennzeichnet, dass** die Produkte aus der abgehenden Lösung, Suspension, Reaktionsmischung, Kultur oder einem Äquivalent (8) gesammelt und aus dem Bioreaktor entfernt werden.

## Revendications

1. Procédé destiné à réaliser une fermentation dans un bioréacteur à agitation par circulation d'air en amenant deux ou plusieurs gaz différents dans le bioréacteur à agitation par circulation d'air de sorte que le bioréacteur ait différents compartiments qui possèdent des parois médianes imperméables ou partiellement perméables entre eux afin de former une partie aérobie et une partie anaérobie, la partie aérobie étant localisée à l'intérieur de la partie anaérobie du bioréacteur ;
**caractérisé en ce que** les différents gaz sont amenés dans les différentes parties du bioréacteur, les différents gaz incluant :
a) un mélange de gaz sans oxygène (4b, 5b) qui est amené dans la partie anaérobie du bioréacteur, dans lequel le mélange de gaz contient un gaz inerte pour évacuer ou pour collecter les produits microbiens d'évaporation ou autres de réaction cellulaire ou substances de déchet ou autres substances d'évaporation, afin d'augmenter le rendement ou la productivité de fermentation anaérobie ou microaérobie avec ledit gaz inerte, et **en ce que**
b) de l'air ou autre gaz contenant de l'oxygène (4a, 5a) qui est amené dans la partie aérobie du bioréacteur, dans lequel les gaz amenés dans le réacteur provoquent des interphases entre les phases liquide et gazeuse, et
dans lequel le flux des gaz amenés dans le bioréacteur est ajusté grâce à l'utilisation d'une unité de commande, qui permet d'ajuster les conditions à l'intérieur du bioréacteur en fonction des données de mesure de capteur obtenues à partir des concentrations ou caractéristiques des substances sortant du bioréacteur, et
dans lequel le mélange de réactifs dans le bioréacteur est réalisé par l'air, le gaz sans oxygène ou le gaz contenant de l'oxygène, en évitant une agitation mécanique.

2. Procédé selon la revendication 1, **caractérisé en ce que** le gaz inerte est l'azote.

3. Procédé selon la revendication 1 ou 2, **caractérisé en ce que** tous les gaz (5a, 5b, 4a, 4b) amenés au bioréacteur ou une partie de ceux-ci sont stérilisés.

4. Procédé selon n'importe laquelle des revendications 1 à 3, **caractérisé en ce que** les gaz (5a, 5b, 4a, 4b) amenés au réacteur forment des bulles dans la solution, la suspension, le mélange de réaction, la culture ou équivalent (3) inclus dans le bioréacteur.

5. Procédé selon n'importe laquelle des revendications 1 à 4, **caractérisé en ce que** les substances libérées sous une forme gazeuse à partir de la solution, la suspension, le mélange de réaction, la culture ou équivalent (3) sont amenées en sortie en tant que mélange de gaz uniforme, dont la composition peut varier.

6. Procédé selon n'importe laquelle des revendications 1 à 5, **caractérisé en ce que** les substances libérées sous une forme gazeuse à partir de la solution, la suspension, le mélange de réaction, la culture ou équivalent (3) sont amenées en sortie en tant deux ou plusieurs mélanges de gaz avec différentes compositions en utilisant deux ou plusieurs trajets différents.

7. Procédé selon la revendication 5 ou 6, **caractérisé en ce que** des substances gazeuses, liquides ou solides sont collectées pour une exploitation à partir des un ou plusieurs flux de gaz issus du bioréacteur.

8. Procédé selon n'importe laquelle des revendications 1 à 7, **caractérisé en ce que** les conditions physiques, température ou pression, des gaz (5a, 5b, 4a, 4b) amenées dans le bioréacteur à partir de deux ou plusieurs sources sont différentes lorsqu'elles sont comparées les unes aux autres.

9. Procédé selon n'importe laquelle des revendications 1 à 8, **caractérisé en ce que** des substances sont ajoutées à ou enlevées de la solution, la suspension, le mélange de réaction, la culture ou équivalent (3) de manière continue.

10. Procédé selon n'importe laquelle des revendications 1 à 9, **caractérisé en ce que** des bactéries sont utilisées en tant que biocatalyseurs dans le bioréacteur.

11. Procédé selon n'importe laquelle des revendications 1 à 10, **caractérisé en ce que** facultativement des bactéries anaérobies sont utilisées en tant qu'organismes de production dans la fermentation dans le bioréacteur.

12. Procédé selon la revendication 11, **caractérisé en ce que** des bactéries entériques ou des entérobactéries sont utilisées en tant qu'organismes de production.

13. Procédé selon la revendication 12, **caractérisé en ce que** les organismes de production appartiennent aux genres *Klebsiella* ou *Enterobacter* ou n'importe quels autres genres utilisant la fermentation butanediolique dans leur métabolisme.

14. Procédé selon n'importe laquelle des revendications 11 à 13, **caractérisé en ce que** la bactérie utilisée en tant qu'organisme de production est apte à fixer l'azote de l'atmosphère dans la partie aérobie du bioréacteur, et l'azote de l'atmosphère peut ainsi être exploité également dans la fermentation anaérobie.

15. Bioréacteur à agitation par circulation d'air pour réaliser une fermentation selon n'importe laquelle des revendications 1 à 14, le bioréacteur comprenant une cuve ou une chambre de réaction (1), qui est configurée pour avoir différents compartiments qui possèdent des parois médianes imperméables ou partiellement perméables entre eux afin de fournir une partie aérobie du bioréacteur et une partie anaérobie du bioréacteur, dans lequel la partie aérobie est localisée à l'intérieur de la partie anaérobie du bioréacteur, **caractérisé en ce que** le bioréacteur est configuré de telle sorte que différents gaz sont amenés dans les différentes parties du bioréacteur, les différents gaz incluant a) un mélange de gaz sans oxygène (4b, 5b) qui est amené dans la partie anaérobie du bioréacteur, et b) de l'air ou autre gaz contenant de l'oxygène (4a, 5a) qui est amené dans la partie aérobie du bioréacteur, et **en ce que** le bioréacteur est raccordé à une unité de commande permettant un ajustement des conditions à l'intérieur du bioréacteur en fonction des données de mesure de capteur obtenues à partir des concentrations ou caractéristiques des substances sortant du bioréacteur, dans lequel le bioréacteur ne contient pas de moyens mécaniques pour l'agitation.

16. Appareil selon la revendication 15, **caractérisé en ce que** les gaz (5a, 5b, 4a, 4b) sont amenés entièrement ou partiellement dans la solution, la suspension, le mélange de réaction, la culture ou équivalent (3) dans le bioréacteur.

17. Appareil selon n'importe laquelle des revendications 15 à 16, **caractérisé en ce que** les gaz sortants en provenance du bioréacteur ou de la chambre de réaction, et avec eux les liquides libérés, sont collectés en tant que produits utilisables.

18. Appareil selon n'importe laquelle des revendications 15 à 17, **caractérisé en ce que** les produits sont collectés à partir de la solution, la suspension, le mélange de réaction, la culture ou équivalent (8) en sortie et sont enlevés du bioréacteur.
